# EUROPÄISCHE PATENTSCHRIFT

(11) **EP 3 219 353 B1**
(45) Veröffentlichungstag und Bekanntmachung des Hinweises auf die Patenterteilung: **09.12.2020**
(21) Anmeldenummer: 16193231.4
(22) Anmeldetag: 11.10.2016
(51) Int. Cl.: A61M 39/10, F16L 37/098

(54) **VERBINDUNGSVORRICHTUNG, INJEKTIONSVORRICHTUNG ZUR INJEKTION EINER MEDIZINISCHEN FLÜSSIGKEIT UND VERFAHREN ZUM BETRIEB EINER SOLCHEN INJEKTIONSVORRICHTUNG**
CONNECTING DEVICE, INJECTION DEVICE FOR INJECTING A MEDICAL LIQUID, AND METHOD FOR OPERATING SUCH AN INJECTION DEVICE
DISPOSITIF DE LIAISON, DISPOSITIF D'INJECTION D'UN LIQUIDE MÉDICAL ET PROCÉDÉ DE FONCTIONNEMENT D'UN TEL DISPOSITIF D'INJECTION

(30) Priorität: 17.03.2016 DE 102016104937
(43) Veröffentlichungstag der Anmeldung: 20.09.2017
(73) Patentinhaber: Ulrich GmbH & Co. KG, 89081 Ulm (DE)
(72) Erfinder: Striggow, Uwe, 72631 Aichtal (DE); Seibold, Felix, 89081 Ulm (DE); Götz, Markus, 89134 Blaustein (DE)
(74) Vertreter: Charrier Rapp & Liebau

(56) Entgegenhaltungen:
- EP-A2- 2 669 564
- WO-A1-2009/051669
- WO-A2-01/41849
- WO-A2-2014/207385
- AU-A1- 2011 214 832
- DE-U1-202015 102 734

## Beschreibung

Die Erfindung betrifft eine Verbindungsvorrichtung zur Verbindung einer medizinischen Schlauchleitung mit einer medizinischen Flüssigkeitstransfereinrichtung nach dem Oberbegriff des Anspruchs 1 sowie eine Injektionsvorrichtung zur Injektion einer medizinischen Flüssigkeit und ein Verfahren nach dem Obergegriff von Anspruch 8.

Die intravenöse Injektion von medizinischen Flüssigkeiten in den Körper eines Menschen ist ein risikobehafteter Eingriff, der eine große Sorgfalt und die strikte Einhaltung hygienischer Regeln erfordert. Zur Vermeidung von Fehlern in diesem Bereich ist bereits die Verwendung von RFID-Transpondern vorgeschlagen worden. So beschreibt die EP 2 170 428 A1 den Einsatz von RFID-Transpondern bei einem Dialysegerät. Die Anmeldung AU2011214832 beschreibt ebenfalls den Einsatz von RFID-Transpondern, bei Konnektoren. Bei der Dialyse wird ein Gemisch aus mehreren verschiedenen Flüssigkeiten in die venöse Blutbahn eines Patienten injiziert, wobei die Mischung erst unmittelbar vor der Injektion in einem Dialysegerät erfolgt. Um Verwechslungen zu vermeiden, ist in dem bekannten Dialysegerät vorgesehen, an Schlauchleitungen, welche mit Flüssigkeitsbehältern verbunden sind, und an einer austauschbar in dem Dialysegerät einsetzbaren Kassette, in der die Mischung der verschiedenen Flüssigkeiten aus den Flüssigkeitsbehältern erfolgt, jeweils RFID-Transponder anzubringen, die von einer Leseeinheit des Dialysegerätes gelesen werden können. Dabei sind mehrere Antennen der Leseeinheit in einen automatischen Verbindungsmechanismus integriert, welcher die Schlauchleitungen der Flüssigkeitsbehälter mit der Kassette mikrocomputergesteuert verbindet und trennt.

Der Erfindung liegt die Aufgabe zugrunde, eine Verbindungsvorrichtung zur Verbindung einer medizinischen Schlauchleitung mit einer medizinischen Flüssigkeitstransfereinrichtung und eine Injektionsvorrichtung zu schaffen, die mit geringem apparativem Aufwand eine Überwachung der medizinisch korrekten Verwendung der verbundenen Komponenten erlaubt, und ein zweckmäßiges Verfahren zum Betrieb einer Injektionsvorrichtung unter Verwendung einer solchen Verbindungsvorrichtung anzugeben.

Gelöst wird diese Aufgabe erfindungsgemäß durch eine Verbindungsvorrichtung mit den Merkmalen des Anspruchs 1, durch eine Injektionsvorrichtung zur Injektion einer medizinischen Flüssigkeit mit den Merkmalen des Anspruchs 5 , sowie durch ein Verfahren mit den Merkmalen des Anspruchs 8 . Bevorzugte Ausführungsformen sind den jeweiligen abhängigen Ansprüchen zu entnehmen.

Bei der erfindungsgemäßen Verbindungsvorrichtung zur Verbindung einer medizinischen Schlauchleitung mit einer medizinischen Flüssigkeitstransfereinrichtung ist ein Steckerteil mit einem ersten Flüssigkeitskanal und ein Kupplungsteil mit einem zweiten Flüssigkeitskanal vorgesehen, wobei das Steckerteil an der Schlauchleitung und das Kupplungsteil an der Flüssigkeitstransfereinrichtung angeordnet ist und das Steckerteil zur Herstellung eines die Flüssigkeitskanäle miteinander verbindenden Durchflussweges für eine medizinische Flüssigkeit mit dem Kupplungsteil koppelbar ist. In dem Steckerteil ist dabei erfindungsgemäß ein erster passiver Transponder integriert und in der Flüssigkeitstransfereinrichtung ist ein zweiter passiver Transponder integriert, wobei der Abstand zwischen dem ersten Transponder und dem zweiten Transponder unterhalb eines vorbestimmten Grenzwertes liegt, der so ausgewählt ist, dass eine Kommunikation eines Lese-/Schreibgerätes mit beiden Transpondern mittels einer einzigen Antenne ermöglich ist, wenn sich das Steckerteil in Eingriff mit dem Kupplungsteil befindet.

Durch den ersten Transponder wird es ermöglicht, den Anschluss einer passenden und korrekten Schlauchleitung an die Flüssigkeitstransfereinrichtung unmittelbar an der Verbindungsvorrichtung unabhängig vom Verlauf der Schlauchleitung abseits des Steckerteils, an dem sie endet, zu überwachen. Wenn das Steckerteil mit dem Kupplungsteil der Flüssigkeitstransfereinrichtung verbunden ist, dann hat es eine definierte Position bezüglich der Flüssigkeitstransfereinrichtung und der in dem Steckerteil integrierte erste Transponder kann durch ein Lese-/Schreibgerät, dessen Antenne an einer Injektionsvorrichtung bezüglich des Kupplungsteils der Flüssigkeitstransfereinrichtung geeignet platziert ist, gelesen und/oder beschrieben werden. Eine Führung für die Schlauchleitung zur geeigneten Positionierung eines an ihr angeordneten Transponders bezüglich einer Antenne eines Lese-/Schreibgerät wird daher nicht benötigt.

Erfindungsgemäß in dem Kupplungsteil ein zweiter passiver Transponder integriert, wodurch eine zusätzliche Überwachung der Verwendung einer passenden und korrekten Flüssigkeitstransfereinrichtung ermöglicht wird. Um beide Transponder ggf. gleichzeitig mit einem Lese-/Schreibgeräts auslesen oder beschreiben zu können, ist der Abstand zwischen dem im Steckerteil integrierten ersten Transponder und dem am Kupplungsteil angeordneten zweiten Transponder unterhalb eines vorbestimmten Grenzwertes so gering, dass eine Kommunikation des Lese-/Schreibgerätes mit beiden Transpondern mittels einer einzigen Antenne möglich ist, wenn sich das Steckerteil in Eingriff mit dem Kupplungsteil befindet. Es kann dann ein Lese-/Schreibgerät mit einer einzigen Antenne eingesetzt werden.

Zweckmäßig ist es, wenn der erste Transponder und der zweite Transponder als RFID-Transponder ausgebildet sind und jeder Transponder einen nur lesbaren ersten Speicher (ROM) mit darin unveränderlich gespeicherten Identifikationsdaten aufweist. Ergänzend dazu kann jeder Transponder auch einen sowohl lesbaren, als auch beschreibbaren zweiten Speicher enthalten. Der nur lesbare Speicher enthält bspw. einen Identifikationscode, anhand dessen ein passendes Originalteil von einem nicht autorisierten oder nicht passenden Teil unterschieden werden kann. Der ggf. ergänzend vorhandene beschreibbare Speicher erlaubt die Speicherung von Daten während der Benutzung des jeweiligen Teils (Schlauchleitung bzw. Flüssigkeitstransfereinrichtung).

Besonders nützlich ist die erfindungsgemäße Ausstattung eines Steckerteils einer medizinischen Schlauchleitung mit einem Transponder dann, wenn die Schlauchleitung an ihrem dem Steckerteil entgegengesetzten Ende einen zum Anschluss einer Kanüle ausgebildeten Verbinder aufweist, also dazu bestimmt ist, unmittelbar zu einem Patienten zu führen, dem intravenös eine Flüssigkeit injiziert werden soll, da in diesem Fall besonders strenge hygienische Anforderungen bestehen, die automatisiert und sicher durch eine elektronische Überwachung überwacht und sicher gestellt werden können. Gleiches gilt auch für eine Flüssigkeitstransfereinrichtung, wenn es sich dabei um eine in eine Injektionsvorrichtung zur intravenösen Injektion einer medizinischen Flüssigkeit in den Körper eines Menschen oder Tieres einsetzbare Kassette handelt, die eine Vielzahl von Eingangsanschlüssen für die Zufuhr von medizinischen Flüssigkeiten und eine Vielzahl von Flüssigkeitskanälen und Ventilen aufweist, und deren Ausgangsanschluss das Kupplungsteil angeordnet ist.

Die erfindungsgemäße Verbindungsvorrichtung wird in eine Injektionsvorrichtung zur intravenösen Injektion einer medizinischen Flüssigkeit eingesetzt. Die Injektionsvorrichtung umfasst dabei ein Gehäuse, eine Fördereinrichtung zur Förderung der Flüssigkeit und eine an wenigstens einen Flüssigkeitsbehälter anschließbare und austauschbar in dem Gehäuse einsetzbare Flüssigkeitstransfereinrichtung, welche das Kupplungsteil und eine daran über das Steckerteil angeschlossene Schlauchleitung umfasst, wobei das Steckerteil zur Herstellung einer Verbindung mit dem Kupplungsteil gekoppelt ist. Gemäß der Erfindung ist dabei in dem Steckerteil ein erster passiver Transponder und/oder in dem Kupplungsteil ein zweiter passiver Transponder integriert.

An dem Gehäuse der Injektionsvorrichtung ist zweckmäßig ein Lese-/Schreibgerät zum Auslesen und/oder Beschreiben des ersten und/oder des zweiten Transponders angeordnet, wobei das Lese-/Schreibgerät aufgrund der benachbarten Anordnung der beiden Transponder nur eine einzige Antenne benötigt, um sowohl den ersten Transponder als auch den zweiten Transponder auslesen oder beschreiben zu können, wenn die Flüssigkeitstransfereinrichtung über die Verbindungsvorrichtung mit der Schlauchleitung gekoppelt ist.. Dadurch können die in dem Steckerteil bzw. in der Flüssigkeitstransfereinrichtung angeordneten Transponder ggf. gleichzeitig ausgelesen bzw. beschrieben werden. Das Lese-/Schreibgerät weist nur eine einzige Antenne auf, über die es mit den beiden Transpondern kommunizieren kann. Ein erfindungsgemäßes Verfahren zum Betrieb einer solchen Injektionsvorrichtung zur intravenösen Injektion einer medizinischen Flüssigkeit in den Körper eines Menschen oder Tieres sieht folgende Schritte vor:
a. Einsetzen der Flüssigkeitstransfereinrichtung in das Gehäuse der Injektionsvorrichtung und Verbinden der Flüssigkeitstransfereinrichtung mit wenigstens einem Flüssigkeitsbehälter,
b. Herstellen einer Verbindung zwischen der Flüssigkeitstransfereinrichtung und der Schlauchleitung über die Verbindungsvorrichtung,
c. Auslesen der in dem ersten Transponder gespeicherten Identifikationsdaten für die Flüssigkeitstransfereinrichtung (2) und der in dem zweiten Transponder gespeicherten Identifikationsdaten für die Schlauchleitung über das Lese-/Schreibgerät.

Bei diesem Verfahren können aus dem ersten Transponder und aus dem zweiten Transponder vor Beginn einer Förderung einer zu injizierenden Flüssigkeit Daten gelesen und abgeglichen werden, wobei mit der Förderung nur dann begonnen wird, wenn die gelesenen Daten in einem vorbestimmten Bereich zulässiger Werte liegen oder mit Referenzdaten übereinstimmen. Hierdurch kann bspw. zuverlässig vermieden werden, dass eine medizinische Flüssigkeit in den Körper eines Patienten injiziert wird, wenn eine Schlauchleitung und/oder eine Flüssigkeitstransfereinrichtung, deren Verwendung hierzu beabsichtigt ist, nicht den einschlägigen Vorschriften entspricht oder vorher schon einmal für eine Injektion verwendet worden ist.

Zweckmäßig ist es hierbei, wenn die in dem im Steckerteil integrierten ersten Transponder gespeicherten Daten einen Identifikationscode enthalten, welcher in einem nur lesbaren Speicher des ersten Transponders gespeichert ist, und/oder wenn die in dem in der Flüssigkeitstransfereinrichtung angeordneten zweiten Transponder gespeicherten Daten einen Identifikationscode enthalten, welcher in einem nur lesbaren Speicher des zweiten Transponders gespeichert ist. Anhand eines solchen Identifikationscodes kann ein zur Verwendung an der Injektionsvorrichtung zugelassenes (neues und steriles) Originalteil (Schlauchleitung bzw. Flüssigkeitstransfereinrichtung) von einem nicht zugelassenen Teil mit beispielsweise zweifelhafter Sterilität oder einem schon einmal verwendeten Teil unterschieden und die Injektion einer Flüssigkeit in einen Patienten unter Verwendung eines nicht zugelassenen Teils unterbunden werden.

Für die Einhaltung einschlägiger Hygienevorschriften ist es besonders vorteilhaft, wenn anhand der vor Beginn einer Förderung einer zu injizierenden Flüssigkeit aus dem ersten und/oder dem zweiten Transponder ausgelesenen Daten ermittelt wird, ob und zu welchem Zeitpunkt zuvor erstmalig eine Förderung einer zu injizierenden Flüssigkeit unter Verwendung der Schlauchleitung bzw. der Flüssigkeitstransfereinrichtung erfolgt ist, und dass mit der Förderung zum aktuellen Zeitpunkt nur dann begonnen wird, wenn die Zeitdifferenz zwischen dem aktuellen Zeitpunkt und dem ausgelesenen Zeitpunkt der erstmaligen Verwendung unterhalb eines vorbestimmten Grenzwertes liegt. Hierbei kann der vorbestimmte Grenzwert der Zeitdifferenz für die Schlauchleitung ein anderer als für die Flüssigkeitstransfereinrichtung sein. Das medizinische Personal, welches eine Injektionsvorrichtung benutzt, wird auf diese Weise von einer manuellen Protokollierung der Verwendungszeit der einzelnen Komponenten entbunden und die Gefahr eines Verstoßes gegen die sich auf die Verwendungszeit beziehenden Hygienevorschriften durch Fehler bei der manuellen Protokollierung wird eliminiert.

Um die Protokollierung der Verwendungsdauer soweit wie möglich zu automatisieren und das medizinische Personal zu entlasten, ist es zweckmäßig, dass der Zeitpunkt der erstmaligen Verwendung der Schlauchleitung in einen Speicher des ersten Transponders geschrieben wird, wenn anhand der vor Beginn einer Förderung einer zu injizierenden Flüssigkeit aus dem ersten Transponder ausgelesenen Daten erkannt wird, dass die Schlauchleitung erstmalig verwendet wird. Entsprechend kann der Zeitpunkt der erstmaligen Verwendung der Flüssigkeitstransfereinrichtung in einen Speicher des zweiten Transponders geschrieben werden, wenn anhand der vor Beginn einer Förderung einer zu injizierenden Flüssigkeit aus dem zweiten Transponder ausgelesenen Daten erkannt wird, dass die Flüssigkeitstransfereinrichtung erstmalig verwendet wird. Ein neues Teil (Schlauchleitung bzw. Flüssigkeitstransfereinrichtung) wird auf diese Weise automatisch mit seinem Inbetriebnahmezeitpunkt gekennzeichnet, ohne dass das medizinische Personal eine entsprechende Information einzugeben braucht.

Vorteilhaft ist es, wenn auf einer Anzeigeeinheit der Injektionsvorrichtung die bisherige Verwendungsdauer und/oder die noch zulässige Verwendungsdauer der Schlauchleitung und/oder der Flüssigkeitstransfereinrichtung angezeigt wird. Das medizinische Personal kann sich hierdurch frühzeitig auf die Notwendigkeit des Einsatzes eines neuen Teils einstellen und dafür sorgen, dass ein solches bereitsteht, sobald es benötigt wird. Ferner kann auf einer Anzeigeeinheit der Injektionsvorrichtung eine Fehlermeldung angezeigt werden, wenn die aus einem Transponder gelesenen Daten außerhalb des vorbestimmten Bereichs zulässiger Werte liegen, um das medizinische Personal über den Grund zu informieren, weshalb ein Injektionsvorgang blockiert worden ist.

Diese und weitere Vorteile und Merkmale der erfindungsgemäßen Verbindungsvorrichtung ergeben sich aus dem nachfolgend unter Bezugnahme auf die begleitenden Zeichnungen näher beschriebenen Ausführungsbeispiel. In den Zeichnungen zeigt
- **Fig. 1**: eine Längsschnittansicht des Kupplungsteils einer erfindungsgemäßen Verbindungsvorrichtung,
- **Fig. 2**: eine Längsschnittansicht des Steckerteils einer erfindungsgemäßen Verbindungsvorrichtung,
- **Fig. 3**: eine Längsschnittansicht einer erfindungsgemäßen Verbindungsvorrichtung mit in das Kupplungsteil eingestecktem Steckerteil und
- **Fig. 4**: eine Darstellung eines erfindungsgemäßen Verfahrens in Form eines Programmablaufplans.

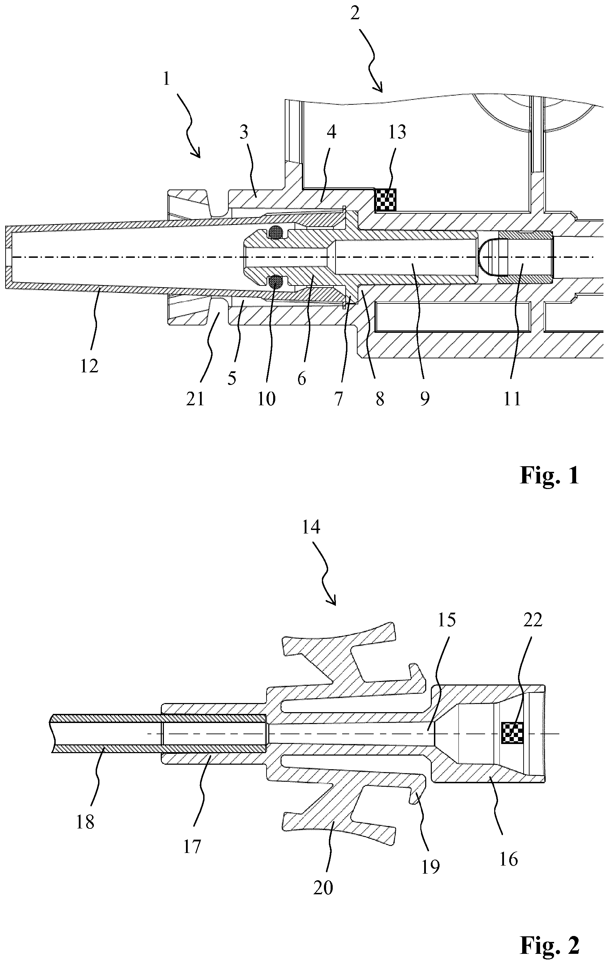

In Fig. 1 ist das Kupplungsteil 1 einer erfindungsgemäßen Verbindungsvorrichtung in einer Längsschnittansicht dargestellt. Es ist ein integraler Bestandteil einer Kassette 2, die austauschbar in ein Gehäuse einer Injektionsvorrichtung zur Injektion von Kontrastmitteln in den Körper eines Menschen oder Tieres einsetzbar ist. Die Kassette 2, von der in Fig. 1 nur ein kleiner Ausschnitt dargestellt ist, enthält eingangsseitig jeweils mehrere Anschlüsse für Flüssigkeitsbehälter, bspw. Behälter für die Kontrastmittel und eine Spüllösung wie NaCl, sowie in einem Kassettengehäuse ausgeformte Flüssigkeitskanäle, die von einer flexiblen Membran abgedeckt sind und von außen durch mechanische Aktoren, welche auf die Membran einwirken, geöffnet und verschlossen werden können. Ausgangsseitig ist an der Kassette 2 ein schlaufenförmig gebogener Pumpenschlauch angeschlossen, der in eine Schlauchpumpe der Injektionsvorrichtung einlegbar ist.

Das Kupplungsteil 1 stellt den Ausgangsanschluss der Kassette 2 dar, der an seinem stromaufwärtigen Ende mit dem stromabwärtigen Ende des Pumpenschlauchs verbunden ist und an seinem stromabwärtigen Ende mit einem Patientenschlauch verbindbar ist, der eine Kanüle zum Einstechen in eine Vene eines Patienten aufweist. Die Kassette 2 bildet somit die Schnittstelle zwischen den Behältern mit den zu injizierenden Flüssigkeiten und dem Patientenschlauch und ermöglicht die Vermeidung eines unmittelbaren Kontaktes der Injektionsvorrichtung mit den zu injizierenden Flüssigkeiten.

Das Kupplungsteil 1 hat im Wesentlichen die Form eines Rohres, das an einer Ecke des Gehäuses der Kassette 2 parallel zu einer ihrer Seiten angeordnet ist. Zweckmäßig ist das Kupplungsteil 1 einteilig am Kassettengehäuse angeformt. Ein äußerer Abschnitt des Kupplungsteils 1 wird durch einen Vorsprung 3 gebildet, der von der Kassette 2 nach außen abragt. Ein innerer Abschnitt des Kupplungsteils 1 wird durch einen zu dem Vorsprung 3 koaxialen Rücksprung 4 im Gehäuse der Kassette 2 gebildet. Durch den Vorsprung 3 und den Rücksprung 4 wird ein Hohlraum 5 geschaffen, der sich in die Kassette 2 hinein erstreckt. In diesem Hohlraum 5 enthält das Kupplungsteil 1 einen Einsatz 6, der ebenfalls im Wesentlichen die Form eines Rohres hat und im mittleren Teil seiner Länge einen Flansch 7 aufweist, mit dem er an einer Schulter 8 des Rücksprungs 4 anliegt und dort bspw. durch eine Klebung befestigt ist. Nahe seinem vorderen, von dem Vorsprung 3 überdeckten Ende trägt der Einsatz 6 in einer Ringnut einen O-Ring 10. In seinem Inneren bildet der Einsatz 6 einen Kanal 9 für die aus der Kassette 2 in Richtung des Patientenschlauches fließende Flüssigkeit. Im Inneren der Kassette 2 befindet sich unmittelbar vor dem hinteren Ende des Einsatzes 6 bevorzugt ein Filter 11, welches dorthin vor dem Einbringen des Einsatzes 6 eingeführt wurde und dort bspw. durch Klebung oder Klemmung befestigt ist.

Im unbenutzten Zustand ist das Kupplungsteil 1, wie in Figur 1 gezeigt, durch eine Kappe 12 abgedeckt, die in den Hohlraum 5 bis zu dem Flansch 7 des Einsatzes 6 eingeschoben und durch Klemmung zwischen dem Rücksprung 4 und dem Einsatz 6 mit dem Kupplungsteil 1 verbunden ist. Zum Anschließen eines Patientenschlauches an die Kassette 2 kann diese Verbindung durch eine ausreichend große axiale Zugkraft gelöst und die Kappe 12 dann in axialer Richtung aus dem Hohlraum 5 entfernt werden.

An der Schulter 8 des Rücksprungs 4 ist an dem Kupplungsteil 1 im Inneren der Kassette 2 ein passiver RFID-Transponder 13 angeordnet, in dem Identifikationsdaten der Kassette 2 gespeichert sind. Zur Kommunikation mit dem Transponder 13 enthält die Injektionsvorrichtung, in der die Kassette 2 verwendet wird, ein mit der Steuerung der Injektionsvorrichtung verbundenes und an einem Gehäuse der Injektionsvorrichtung angeordnetes RFID-Lese-/Schreibgerät. Auf die Funktion des Transponders 13 wird später noch näher eingegangen. Er ist bevorzugt unlösbar mit dem Gehäuse der Kassette 2 verbunden, beispielsweise darin eingegossen oder damit verklebt.

In Fig. 2 ist ein zu dem Kupplungsteil 1 passendes Steckerteil 14 in einer Längsschnittansicht dargestellt. Es ist, wie das Gehäuse der Kassette 2, ein Spritzgussteil aus Kunststoff und hat im Wesentlichen, wie das Kupplungsteil 1, die Form eines Rohres, wobei sein Außendurchmesser etwas kleiner ist als der Innendurchmesser des Hohlraumes 5 des Kupplungsteils 1, damit es dort nach der Entfernung der Kappe 12 in den Hohlraum 5 eingeführt werden kann, um eine Verbindung mit dem Kupplungsteil 1 zum Durchleiten einer Flüssigkeit herzustellen. Im unbenutzten Zustand ist das Steckerteil 14 durch eine in Fig. 2 nicht dargestellte Kappe, deren Form mit derjenigen der Kappe 12 des Kupplungsteils 1 vergleichbar ist, abgedeckt. Diese Kappe umschließt den stromaufwärtigen Abschnitt 16 des Steckerteils 14 im unbenutzten Zustand von außen.

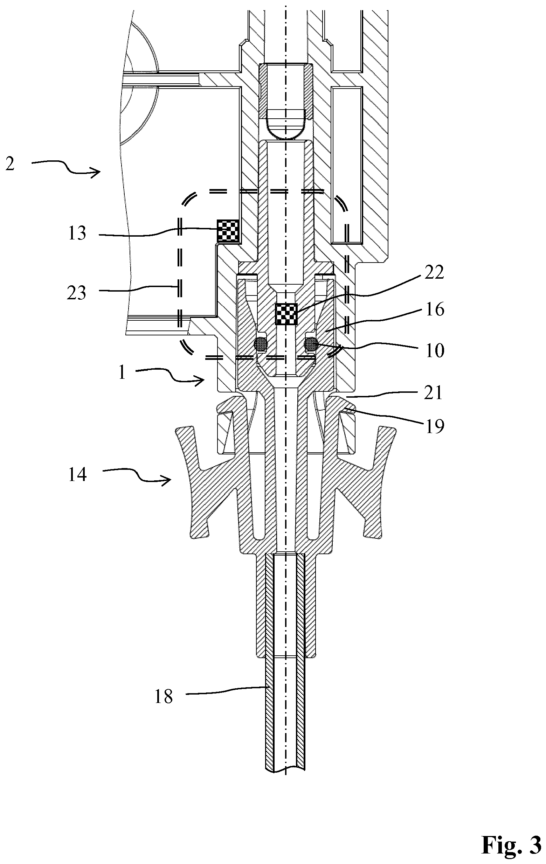

Der durch das Steckerteil 14 in seinem Inneren gebildete Kanal 15 erweitert sich im stromaufwärtigen Abschnitt 16 des Steckerteils 14 trichterförmig. Der stromaufwärtige Abschnitt 16 des Steckerteils 14 ist zum Einführen in das Kupplungsteil 1 bestimmt. Im eingeführten Zustand, der in Figur 3 gezeigt ist, umschließt der Abschnitt 16 des Steckerteils 14 das stromabwärtige Ende des Einsatzes 6 des Kupplungsteils 1 und dichtet mit dem O-Ring 10 ab.. Dies zeigt die Schnittansicht von Fig. 3, in der das Kupplungsteil 1 und das Steckerteil 14 im miteinander verbundenen Zustand dargestellt sind, wobei beide Teile gegenüber den Figuren 1 und 2 um 90° entgegen dem Uhrzeigersinn gedreht sind.

Der stromabwärtige Abschnitt 17 des Steckerteils 14, der im verbundenen Zustand dem Kupplungsteil 1 abgewandt ist, bildet einen Anschlussstutzen für eine Schlauchleitung 18, welche einen Patientenschlauch bildet, durch den der Ausgangsanschluss der Kassette 2 mit einem Patienten verbunden wird. Das eine Ende der Schlauchleitung 18 ist fest mit dem Steckerteil 14 verbunden, bspw. durch Klebung oder Klemmung, und das andere Ende der Schlauchleitung 18 ist mit einer (hier nicht gezeigten) Kanüle verbunden.

In seinem mittleren Teil sind an dem Steckerteil 14 zwei einander diametral gegenüberliegende Schnapphaken 19 mit daran angeformten Griffen 20 vorgesehen, die durch manuellen Druck auf die Griffe 20 radial nach innen bewegt werden können. Dadurch wird das Einführen des Steckerteils 14 in das Kupplungsteil 1 erleichtert.Zum Herausziehen des Steckerteils 14 aus dem Kupplungsteil 1 kann durch Druck auf die Griffe 20 eine Rastverbindung zwischen dem Steckerteil 14 und dem Kupplungsteil 1 gelöst werden. Diese Rastverbindung wird beim Verbinden des Steckerteils 14 mit dem Kupplungsteil 1 durch Zusammenwirken der Schnapphaken 19 mit dazu korrespondierenden Ausschnitten 21 hergestellt, die nahe dem Ende des Vorsprungs 3 in dem Kupplungsteil 1 vorgesehen sind. Die Schnapphaken 19 rasten beim Einführen des Steckerteils 14 in das Kupplungsteil 1 in die diametral einander gegenüberliegenden Ausschnitte 21 ein, um das Steckerteil 14 an dem Kupplungsteil 1 zu fixieren, wenn es bis zum Anschlag in den Hohlraum 5 eingeführt ist. Durch diese Rastverbindung wird ein unbeabsichtigtes Lösen der Verbindung verhindert (Figur 3).

In dem Steckerteil 14 ist ein weiterer RFID-Transponder 22 angeordnet, und zwar im stromaufwärtigen Abschnitt 16, der im verbundenen Zustand der Verbindungsvorrichtung in den Hohlraum 5 im Inneren des Kupplungsteils 1 hineinragt. Der Transponder 22 ist zweckmäßig unlösbar mit dem Steckerteil 14 verbunden, beispielsweise darin eingegossen oder damit verklebt, und enthält in einem Datenspeicher (ROM) Identifikationsdaten zur Identifikation der Schlauchleitung 18. Wie Fig. 3 zeigt, befindet sich der RFID-Transponder 22 des Steckerteils 14 im gekoppelten Zustand im Inneren des Kupplungsteils 1 jenseits des O-Ringes 10 an dem Einsatz 6 und es besteht nur ein geringer Abstand zu dem RFID-Transponder 13 des Kupplungsteils 1. Hierdurch wird es ermöglicht, mit beiden RFID-Transpondern 13 und 22 über eine einzige Antenne 23 eines RFID-Lese-/Schreibgerätes, deren Lage in Fig. 3 gestrichelt dargestellt ist, zu kommunizieren. Dadurch wird es ermöglicht, mit einem in einem Gehäuse einer Injektionsvorrichtung angeordneten Lese-/Schreibgerät mit einer einzigen Antenne die in den Transpondern 22, 22 gespeicherten Identifikationsdaten der Flüssigkeitstransfereinrichtung (Kassette 2) und der daran angeschlossenen Schlauchleitung 18 gleichzeitig auszulesen.

Die RFID-Transponder 13 und 22 ermöglichen dadurch eine Überwachung der korrekten Verwendung der Kassette 2, deren fester Bestandteil das Kupplungsteil 1 ist, und der Schlauchleitung 18, deren fester Bestandteil das Steckerteil 14 ist. So kann durch ein Auslesen eines Identifikationscodes aus einem nur lesbaren Speicher eines RFID-Transponders 13 oder 22 festgestellt werden, ob es sich bei einer Kassette 2 bzw. eine Schlauchleitung 18 um ein vom Hersteller der Injektionsvorrichtung autorisiertes Verbrauchsteil handelt, das für die Verwendung in der Injektionsvorrichtung geeignet ist und den einschlägigen Anforderungen der medizinischen Zulassung genügt.

Des weiteren unterliegen eine Kassette 2 und eine zu einem Patienten führende Schlauchleitung 18 auch hygienischen Vorschriften, welche die Verwendungsdauer begrenzen, wobei die zulässige Verwendungsdauer einer zu einem Patienten führenden Schlauchleitung 18 üblicherweise kürzer ist als diejenige einer Kassette 2. Beispielsweise kann die zulässige Verwendungsdauer eines Patientenschlauchs bei maximal 12 Stunden und diejenige einer Kassette bei maximal 24 Stunden liegen. Die Einhaltung dieser Vorschriften wird erfindungsgemäß dadurch sichergestellt, dass aus einem Speicher eines RFID-Transponders 13 und/oder 22 ein Zeitstempel-Datensatz ausgelesen wird, der den Zeitpunkt des Beginns der Verwendung einer Kassette 2 bzw. einer Schlauchleitung 18 angibt. Dieser Zeitstempel-Datensatz existiert bei einer neuen Kassette 2 bzw. Schlauchleitung 18 noch nicht, d.h. der hierfür in dem Transponder 13 bzw. 22 vorgesehene Speicherbereich hat dann noch einen Inhalt, welcher anzeigt, dass die jeweilige Komponente neu, also noch unbenutzt ist.

Vor jeder Inbetriebnahme der Fördereinrichtung (Schlauchpumpe) der Injektionsvorrichtung wird auch dieser Zeitstempel-Datensatz ausgelesen und durch einen Vergleich mit dem aktuellen Datum und der aktuellen Zeit festgestellt, ob die bisherige Verwendungsdauer noch unter dem maximal zulässigen Wert liegt. Wenn dies der Fall ist, wird der Betrieb der Fördereinrichtung freigegeben, andernfalls eine entsprechende Fehlermeldung auf einer Anzeigeeinheit der Injektionsvorrichtung ausgegeben. Wenn hierbei eine neue Kassette 2 bzw. eine neue Schlauchleitung 18 erkannt wird, dann wird der aktuelle Zeitpunkt als Zeitstempel-Datensatz in den hierfür vorgesehenen Speicher des Transponders 13 bzw. 22 geschrieben und markiert den Beginn der Verwendung.

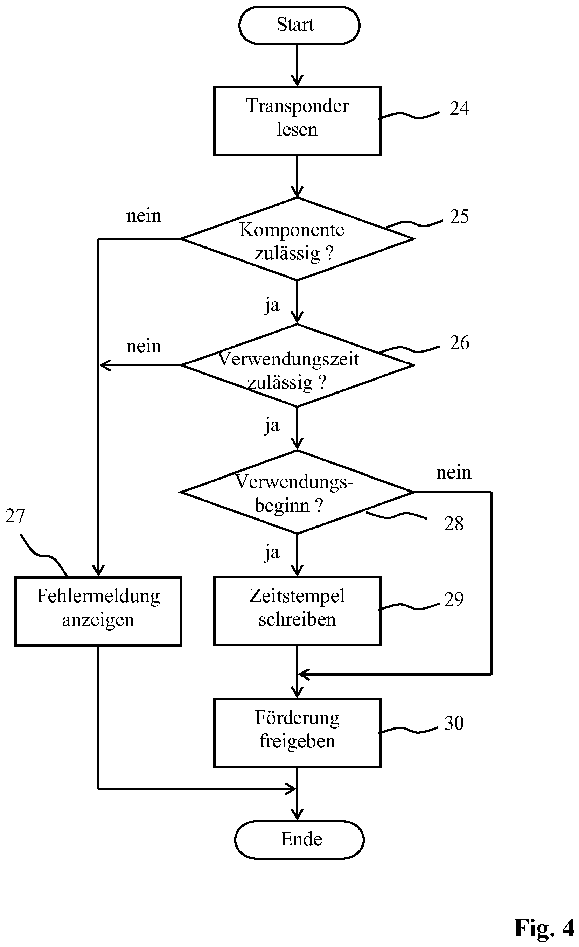

Eine Darstellung dieses Verfahrens, mit dem auch die Einhaltung von Zeitbedingungen für die Verwendung einer Kassette 2 und/oder einer Schlauchleitung erfindungsgemäß überwacht wird, zeigt Fig. 4 in Form eines Programmablaufplans. Als erstes werden in Schritt 24 aus dem Transponder 13 und/oder 22 die relevanten Daten gelesen. Dann wird in Schritt 25 geprüft, ob ein in den ausgelesenen Daten enthaltener Identifikationscode korrekt ist und die Kassette 2 bzw. die Schlauchleitung 18 als zugelassenes Originalteil ausweist. Wenn ja, dann wird in Schritt 26 anhand des ausgelesenen Zeitstempel-Datensatzes durch Vergleich mit dem aus einer Echtzeituhr der Injektionsvorrichtung ausgelesenen aktuellen Zeitpunkt des weiteren geprüft, ob die zulässige Verwendungszeit noch eingehalten ist. Hierbei könnte bei Bedarf auch die voraussichtliche Dauer des Injektionsvorgangs, vor dessen Beginn das erfindungsgemäße Verfahren abläuft, berücksichtigt und die Verwendungszeit bereits als überschritten bewertet werden, wenn sie zwar zum Ausführungszeitpunkt des Prüfschrittes 26 noch nicht überschritten ist, aber während der voraussichtlichen Dauer des anstehenden Injektionsvorgangs, die der Steuerung der Injektionsvorrichtung bekannt ist, überschritten würde.

Wenn einer der Prüfschritte 25 oder 26 ein negatives Ergebnis liefert, dann wird in Schritt 27 eine entsprechende Fehlermeldung angezeigt, die das medizinische Personal über die festgestellte Fehlerursache informiert. Eine Freigabe der Förderung einer Flüssigkeit durch die Pumpe der Injektionsvorrichtung unterbleibt in diesem Fall und der Ablauf des erfindungsgemäßen Verfahrens ist damit abgeschlossen.

Wenn das Ergebnis des Prüfschritts 26 positiv war, wird in Schritt 28 anhand des ausgelesenen Zeitstempel-Datensatzes noch geprüft, ob es sich um den Beginn einer Verwendung der jeweiligen Komponente handelt, d.h. ob die Komponente neu ist. Wenn ja wird noch in Schritt 29 der aktuelle Zeitpunkt als Zeitstempel-Datensatz in einen hierfür vorgesehenen Speicher des Transponders 13 bzw. 22 geschrieben, bevor in Schritt 30 die Förderung einer Flüssigkeit durch die Fördereinrichtung der Injektionsvorrichtung freigegeben wird. Letzteres erfolgt immer dann, wenn beide Prüfschritte 25 und 26 ein positives Ergebnis hatten.

Die Anzeigeeinheit der Injektionsvorrichtung wird auch dazu benutzt, das medizinische Personal über den zeitlichen Status der Schlauchleitung 18 und der Kassette 2 zu informieren, indem dort die bisherige Verwendungsdauer und/oder die zulässige Restdauer der Verwendung angezeigt wird.

In dem Ablaufplan von Fig. 4 nicht dargestellt, aber im Interesse eines Patienten sinnvoll ist die Möglichkeit einer manuellen Aufhebung der Blockierung der Förderung nach Ablauf der zulässigen Verwendungszeit eines Schlauchleitung 18 oder Kassette 2, wenn dieser Ablauf während einer Folge von Injektionen in ein und denselben Patienten eintritt. In diesem Fall ist ein Wechsel der betroffenen Komponente medizinisch nicht notwendig und es wäre sogar für den Patienten eine unnötige Belastung, in einem solchen Fall die Schlauchleitung 18 mit der in den Körper des Patienten eingestochenen Kanüle zu wechseln. Daher ist für diesen Fall die Möglichkeit einer manuellen Freigabe der Förderung durch Eingabe eines entsprechenden Befehls an einer Eingabeeinheit der Injektionsvorrichtung vorgesehen. Die Nutzung der manuellen Freigabe trotz Ablaufs der zulässigen Verwendungszeit liegt in der Verantwortung des medizinischen Personals.

Die in den Figuren 1 bis 3 dargestellten Formen des Kupplungsteils 1 und des Steckerteils 14 sind als beispielhaft zu verstehen. Dem einschlägigen Fachmann ist klar, dass eine erfindungsgemäße Verbindungsvorrichtung auch einen anderen mechanischen Aufbau haben könnte, und dass das erfindungsgemäße Verfahren grundsätzlich nur das Vorhandensein eines Transponders an einem Steckerteil oder einem Kupplungsteil einer medizinischen Verbindungsvorrichtung und keine spezielle Gestalt derselben voraussetzt.

## Patentansprüche

1. Verbindungsvorrichtung zur Verbindung einer medizinischen Schlauchleitung (18) mit einer medizinischen Flüssigkeitstransfereinrichtung (2), wobei die Schlauchleitung (18) ein Steckerteil (14) mit einem ersten Flüssigkeitskanal (15) und die Flüssigkeitstransfereinrichtung (2) ein Kupplungsteil (1) mit einem zweiten Flüssigkeitskanal (9) aufweist und das Steckerteil (14) zur Herstellung eines die Flüssigkeitskanäle (15; 9) miteinander verbindenden Durchflussweges für eine medizinische Flüssigkeit in Eingriff mit dem Kupplungsteil (1) versetzbar ist, wobei in dem Steckerteil (14) ein erster passiver Transponder (22) integriert ist, der gespeicherte Identifikationsdaten für die Schlauchleitung (18) enthält, und in dem Kupplungsteil (1) ein zweiter passiver Transponder (13) integriert ist, der gespeicherte Identifikationsdaten für die Flüssigkeitstransfereinrichtung (2) enthält, und der Abstand zwischen dem ersten Transponder (22) und dem zweiten Transponder (13) unterhalb eines vorbestimmten Grenzwertes liegt, der so ausgewählt ist, dass eine Kommunikation eines Lese-/Schreibgerätes mit beiden Transpondern (22; 13) mittels einer einzigen Antenne (23) ermöglicht ist, wenn sich das Steckerteil (14) in Eingriff mit dem Kupplungsteil (1) befindet, **dadurch gekennzeichnet, dass** die Flüssigkeitstransfereinrichtung (2) eine in eine Injektionsvorrichtung einsetzbare Kassette (2) ist, die eine Vielzahl von Eingangsanschlüssen für die Zufuhr von medizinischen Flüssigkeiten und eine Vielzahl von Flüssigkeitskanälen und Ventilen sowie einen von dem Kupplungsteil (1) gebildeten Ausgangsanschluss aufweist und dass anhand der vor Beginn einer Förderung der zu injizierenden Flüssigkeit aus dem Speicher des ersten Transponder (22) und/oder des zweiten Transponders (13) mittels des Lese-/Schreibgerätes ausgelesenen Daten ermittelt wird, ob und ggf. zu welchem Zeitpunkt zuvor erstmalig eine Förderung einer zu injizierenden Flüssigkeit unter Verwendung der Schlauchleitung (18) bzw. der Flüssigkeitstransfereinrichtung (2) erfolgt ist und dass mit der Förderung zum aktuellen Zeitpunkt nur dann begonnen wird, wenn die Zeitdifferenz zwischen dem aktuellen Zeitpunkt und dem ausgelesenen Zeitpunkt der erstmaligen Verwendung unterhalb eines vorbestimmten Grenzwertes liegt.

2. Verbindungsvorrichtung nach Anspruch 1, **dadurch gekennzeichnet, dass** es sich bei dem ersten Transponder (22) und/oder dem zweiten Transponder (13) um einen RFID-Transponder handelt.

3. Verbindungsvorrichtung nach einem der Ansprüche 1 oder 2, **dadurch gekennzeichnet, dass** der erste Transponder (22) und/oder der zweite Transponder (22) einen nur lesbaren ersten Speicher (ROM), mit einem darin unveränderlich abgespeicherten Identifikationscode und einen sowohl lesbaren, als auch beschreibbaren zweiten Speicher aufweist.

4. Verbindungsvorrichtung nach einem der Ansprüche 1 bis 3, **dadurch gekennzeichnet, dass** das Steckerteil (14) an einem Ende der Schlauchleitung (18) angeordnet ist und die Schlauchleitung (18) an ihrem anderen Ende einen zum Anschluss einer Kanüle vorgerichteten Verbinder aufweist.

5. Injektionsvorrichtung zur Injektion einer medizinischen Flüssigkeit, umfassend ein Gehäuse, eine Fördereinrichtung zur Förderung der Flüssigkeit sowie eine an wenigstens einen Flüssigkeitsbehälter anschließbare und austauschbar in dem Gehäuse einsetzbare Flüssigkeitstransfereinrichtung (2) und eine daran angeschlossene Schlauchleitung (18), **dadurch gekennzeichnet, dass** die Flüssigkeitstransfereinrichtung (2) als eine in die Injektionsvorrichtung einsetzbare Kassette (2) ausgebildet und mit der Schlauchleitung (18) über eine Verbindungsvorrichtung nach einem der voranstehenden Ansprüche verbunden ist.

6. Injektionsvorrichtung nach Anspruch 5, **dadurch gekennzeichnet, dass** an dem Gehäuse der Injektionsvorrichtung ein Lese-/Schreibgerät zum Auslesen und/oder Beschreiben des ersten Transponders (22) und/oder des zweiten Transponders (13) angeordnet ist, wobei das Lese-/Schreibgerät eine einzige Antenne (23) aufweist, über welche sowohl der erste Transponder (22) als auch der zweite Transponder (13) auslesbar und/oder beschreibbar ist.

7. Injektionsvorrichtung nach Anspruch 5 oder 6, **dadurch gekennzeichnet, dass** die in dem ersten Transponder (22) gespeicherten Daten einen Identifikationscode zur Identifikation der Schlauchleitung (18) enthalten, welcher in einem nur lesbaren Speicher des ersten Transponders (22) gespeichert ist, und dass die in dem zweiten Transponder (13) gespeicherten Daten einen Identifikationscode zur Identifikation der Flüssigkeitstransfereinrichtung (2) enthalten, welcher in einem nur lesbaren Speicher des zweiten Transponders (13) gespeichert ist.

8. Verfahren zum Betrieb einer Injektionsvorrichtung nach einem der Ansprüche 5 bis 7, **gekennzeichnet durch** folgende Schritte:
a. Einsetzen der Flüssigkeitstransfereinrichtung (2) in das Gehäuse der Injektionsvorrichtung und Verbinden der Flüssigkeitstransfereinrichtung (2) mit wenigstens einem Flüssigkeitsbehälter,
b. Herstellen einer Verbindung zwischen der Flüssigkeitstransfereinrichtung (2) und der Schlauchleitung (18) über die Verbindungsvorrichtung,
c. Auslesen der in dem ersten Transponder (22) gespeicherten Identifikationsdaten für die Schlauchleitung (18) und der in dem zweiten Transponder (13) gespeicherten Identifikationsdaten für die Flüssigkeitstransfereinrichtung (2) über das Lese-/Schreibgerät, **dadurch gekennzeichnet,** dass anhand der vor Beginn einer Förderung der zu injizierenden Flüssigkeit aus dem Speicher des ersten Transponders (22) und des zweiten Transponders (13) ausgelesenen Daten ermittelt wird, ob und ggf. zu welchem Zeitpunkt zuvor erstmalig eine Förderung einer zu injizierenden Flüssigkeit unter Verwendung der Schlauchleitung (18) bzw. der Flüssigkeitstransfereinrichtung (2) erfolgt ist, und dass mit der Förderung zum aktuellen Zeitpunkt nur dann begonnen wird, wenn die Zeitdifferenz zwischen dem aktuellen Zeitpunkt und dem ausgelesenen Zeitpunkt der erstmaligen Verwendung unterhalb eines vorbestimmten Grenzwertes liegt.

9. Verfahren nach Anspruch 8, **dadurch gekennzeichnet, dass** aus dem ersten Transponder (22) und/oder aus dem zweiten Transponder (13) vor Beginn einer Förderung einer zu injizierenden Flüssigkeit Daten gelesen und die gelesenen Daten mit Referenzdaten verglichen werden, wobei mit der Förderung der zu injizierenden Flüssigkeit nur dann begonnen wird, wenn die gelesenen Daten mit den Referenzdaten zumindest in einem vorbestimmten Bereich übereinstimmen.

10. Verfahren nach Anspruch 8, **dadurch gekennzeichnet, dass** der vorbestimmte Grenzwert der Zeitdifferenz für die Schlauchleitung (18) ein anderer ist als für die Flüssigkeitstransfereinrichtung (2), wobei der Grenzwert für die Schlauchleitung (18) bevorzugt kleiner als der Grenzwert für die Flüssigkeitstransfereinrichtung (2) ist.

11. Verfahren nach Anspruch 8 oder 10, **dadurch gekennzeichnet, dass** der Zeitpunkt einer erstmaligen Verwendung der Schlauchleitung (18) in einen Speicher des ersten Transponders (22) geschrieben wird, wenn anhand der vor Beginn einer Förderung einer zu injizierenden Flüssigkeit aus dem ersten Transponder (22) ausgelesenen Daten erkannt wird, dass die Schlauchleitung (18) erstmalig verwendet wird, und/oder dass der Zeitpunkt der erstmaligen Verwendung der Flüssigkeitstransfereinrichtung (2) in einen Speicher des zweiten Transponders (13) geschrieben wird, wenn anhand der vor Beginn einer Förderung einer zu injizierenden Flüssigkeit aus dem zweiten Transponder ausgelesenen Daten erkannt wird, dass die Flüssigkeitstransfereinrichtung (1) erstmalig verwendet wird.

12. Verfahren nach einem der Ansprüche 8 bis 11, **dadurch gekennzeichnet, dass** auf einer Anzeigeeinheit der Injektionsvorrichtung eine bisherige Verwendungsdauer und/oder eine noch zulässige restliche Verwendungsdauer der eingesetzten Flüssigkeitstransfereinrichtung (2) und/oder der daran angeschlossenen Schlauchleitung (18) angezeigt wird, und/oder dass auf einer Anzeigeeinheit der Injektionsvorrichtung eine Fehlermeldung angezeigt wird, wenn die aus dem ersten und/oder dem zweiten Transponder (22; 13) gelesenen Daten außerhalb eines vorbestimmten Bereichs zulässiger Werte liegen oder nicht in Übereinstimmung mit Referenzwerten sind.

## Claims

1. Connecting device for connecting medical tubing (18) to a medical liquid transfer device (2), wherein the tubing (18) has a plug-in part (14) with a first liquid channel (15) and the liquid transfer device (2) has a coupling part (1) with a second liquid channel (9) and the plug-in part (14) is movable into engagement with the coupling part (1) to produce a throughflow path connecting the liquid channels (15; 9) to one another for a medical liquid, wherein a first passive transponder (22) is integrated in the plug-in part (14) and contains stored identification data for the tubing (18), and a second passive transponder (13) is integrated in the coupling part (1) and contains stored identification data for the liquid transfer device (2), and the distance between the first transponder (22) and the second transponder (13) lies below a predetermined limiting value which is selected so that communication of a read/write device with both transponders (22; 13) is facilitated by means of a single antenna (23) if the plug-in part (14) is engaged with the coupling part (1), **characterised in that** the liquid transfer device (2) is a cassette (2) which is insertable into an injection device and has a plurality of input ports for the supply of medical liquids and a plurality of liquid channels and valves and an output port formed by the coupling part (1) and **in that** using the data read from the memory of the first transponder (22) and/or the second transponder (13) by means of the read/write device before the start of delivery of the liquid to be injected, it is ascertained whether and optionally at which point in time delivery of a liquid to be injected has been effected previously for the first time using the tubing (18) or the liquid transfer device (2) and **in that** delivery at the current point in time is started only if the time difference between the current point in time and the read point in time of first-time use lies below a predetermined limiting value.

2. Connecting device according to claim 1, **characterised in that** the first transponder (22) and/or the second transponder (13) is an RFID transponder.

3. Connecting device according to one of claims 1 or 2, **characterised in that** the first transponder (22) and/or the second transponder (22) has a read-only first memory (ROM) with an unchangeable identification code stored therein and a both readable and writable second memory.

4. Connecting device according to one of claims 1 to 3, **characterised in that** the plug-in part (14) is arranged on one end of the tubing (18) and the tubing (18) at its other end has a connecter prepared for connecting a cannula.

5. Injection device for injection of a medical liquid comprising a housing, a delivery device for delivering the liquid and a liquid transfer device (2), which is connectable to at least one liquid container and is insertable in the housing to be replaceable, and tubing (18) connected thereto, **characterised in that** the liquid transfer device (2) is designed as a cassette (2) which is insertable into the injection device and is connected to the tubing (18) via a connecting device according to one of the preceding claims.

6. Injection device according to claim 5, **characterised in that** a read/write device for reading and/or writing the first transponder (22) and/or the second transponder (13) is arranged on the housing of the injection device, wherein the read/write device has a single antenna (23), via which both the first transponder (22) and the second transponder (13) can be read and/or written.

7. Injection device according to claim 5 or 6, **characterised in that** the data stored in the first transponder (22) contain an identification code for identification of the tubing (18) which is stored in a read-only memory of the first transponder (22), and **in that** the data stored in the second transponder (13) contain an identification code for identification of the liquid transfer device (2) which is stored in a read-only memory of the second transponder (13).

8. Method for operating an injection device according to one of claims 5 to 7, **characterised by** the following steps:
a. inserting the liquid transfer device (2) into the housing of the injection device and connecting the liquid transfer device (2) to at least one liquid container,
b. producing a connection between the liquid transfer device (2) and the tubing (18) via the connecting device,
c. reading the identification data stored in the first transponder (22) for the tubing (18) and the identification data stored in the second transponder (13) for the liquid transfer device (2) via the read/write device, **characterised in that** using the data read from the memory of the first transponder (22) and the second transponder (13) before the start of delivery of the liquid to be injected, it is ascertained whether and optionally at which point in time delivery of a liquid to be injected has been effected previously for the first time using the tubing (18) or the liquid transfer device (2), and **in that** delivery at the current point in time is started only if the time difference between the current point in time and the read point in time of first-time use lies below a predetermined limiting value.

9. Method according to claim 8, **characterised in that** data read from the first transponder (22) and/or from the second transponder (13) before the start of delivery of a liquid to be injected and the read data are compared with reference data, wherein delivery of the liquid to be injected is started only if the read data agree with the reference data at least in a predetermined range.

10. Method according to claim 8, **characterised in that** the predetermined limiting value of the time difference for the tubing (18) is one different than for the liquid transfer device (2), wherein the limiting value for the tubing (18) is preferably smaller than the limiting value for the liquid transfer device (2).

11. Method according to claim 8 or 10, **characterised in that** the point in time of first-time use of the tubing (18) is written into a memory of the first transponder (22) if using the data read from the first transponder (22) before the start of delivery of a liquid to be injected, it is detected that the tubing (18) is being used for the first time, and/or **in that** the point in time of first-time use of the liquid transfer device (2) is written into a memory of the second transponder (13) if using the data read from the second transponder before the start of delivery of a liquid to be injected, it is detected that the liquid transfer device (1) is being used for the first time.

12. Method according to one of claims 8 to 11, **characterised in that** a current use period and/or a still admissible remaining use period of the liquid transfer device (2) used and/or the tubing (18) connected thereto is indicated on a display unit of the injection device, and/or **in that** an error message is displayed on a display unit of the injection device if the data read from the first and/or the second transponder (22; 13) lie outside a predetermined range of admissible values or do not agree with reference values.

## Revendications

1. Dispositif de raccordement pour raccorder une conduite en tuyau flexible (18) médicale à un système de transfert de liquide (2) médical, dans lequel la conduite en tuyau flexible (18) présente une partie enfichable (14) avec un premier canal de liquide (15) et le système de transfert de liquide (2) présente une partie de couplage (1) avec un deuxième canal de liquide (9) et la partie enfichable (14) peut être amenée en prise avec la partie de couplage (1) pour établir une voie de passage, reliant entre eux les canaux de liquide (15 ; 9), pour un liquide médical, dans lequel est intégré dans la partie enfichable (14) un premier transpondeur (22) passif, qui contient des données d'identification mémorisées pour la conduite en tuyau flexible (18) et est intégré dans la partie de couplage (1) un deuxième transpondeur passif (13), qui contient des données d'identification mémorisées pour le système de transfert de liquide (2), et la distance entre le premier transpondeur (22) et le deuxième transpondeur (13) est inférieure à une valeur limite prédéfinie, qui est choisie de telle sorte qu'une communication entre un appareil de lecture/écriture et les deux transpondeurs (22 ; 13) est rendue possible au moyen d'une unique antenne (23) quand la partie enfichable (14) se trouve en prise avec la partie de couplage (1), **caractérisé en ce que** le système de transfert de liquide (2) est une cassette (2) pouvant être insérée dans un dispositif d'injection, qui présente une pluralité de raccords d'entrée pour l'amenée de liquides médicaux et une pluralité de canaux de liquide et de soupapes ainsi qu'un raccord de sortie formé par la partie de couplage (1), et qu'il est déterminé à l'aide des données lues, avant le début d'un refoulement du liquide à injecter, depuis la mémoire du premier transpondeur (22) et/ou du deuxième transpondeur (13) au moyen de l'appareil de lecture/d'écriture si et éventuellement à quel moment un refoulement d'un liquide à injecter a eu lieu au préalable pour la première fois en utilisant la conduite en tuyau flexible (18) ou le système de transfert de liquide (2), et que le refoulement débute au moment instantané seulement quand la différence de temps entre le moment instantané et le moment lu de la toute première utilisation est inférieure à une valeur limite prédéfinie.

2. Dispositif de raccordement selon la revendication 1, **caractérisé en ce que** le premier transpondeur (22) et/ou le deuxième transpondeur (13) sont un transpondeur RFID.

3. Dispositif de raccordement selon l'une quelconque des revendications 1 ou 2, **caractérisé en ce que** le premier transpondeur (22) et/ou le deuxième transpondeur (22) présentent une première mémoire (ROM) seulement lisible avec un code d'identification sauvegardé de manière immuable dans celle-ci et une deuxième mémoire à la fois lisible et inscriptible.

4. Dispositif de raccordement selon l'une quelconque des revendications 1 à 3, **caractérisé en ce que** la partie enfichable (14) est disposée au niveau d'une extrémité de la conduite en tuyau flexible (18), et la conduite en tuyau flexible (18) présente au niveau de son autre extrémité un raccordement prévu pour la connexion d'une canule.

5. Dispositif d'injection pour l'injection d'un liquide médical, comprenant un boîtier, un système de refoulement pour le refoulement du liquide ainsi qu'un système de transfert de liquide (2) pouvant être connecté à au moins un contenant de liquide et pouvant être inséré de manière interchangeable dans le boîtier et une conduite en tuyau flexible (18) connectée à celui-ci, **caractérisé en ce que** le système de transfert de liquide (2) est réalisé en tant qu'une cassette (2) pouvant être insérée dans le dispositif d'injection et est relié à la conduite en tuyau flexible (18) par l'intermédiaire d'un dispositif de raccordement selon l'une quelconque des revendications précédentes.

6. Dispositif d'injection selon la revendication 5, **caractérisé en ce qu'**un appareil de lecture/d'écriture pour lire et/ou inscrire le premier transpondeur (22) et/ou le deuxième transpondeur (13) est disposé au niveau du boîtier du dispositif d'injection, dans lequel l'appareil de lecture/d'écriture présente une unique antenne (23), par l'intermédiaire de laquelle à la fois le premier transpondeur (22) et le deuxième transpondeur (13) peuvent être lus et/ou inscrits.

7. Dispositif d'injection selon la revendication 5 ou 6, **caractérisé en ce que** les données mémorisées dans le premier transpondeur (22) contiennent un code d'identification pour identifier la conduite en tuyau flexible (18), lequel est mémorisé dans une mémoire seulement lisible du premier transpondeur (22), et que les données mémorisées dans le deuxième transpondeur (13) contiennent un code d'identification pour l'identification du système de transfert de liquide (2), lequel est mémorisé dans une mémoire seulement lisible du deuxième transpondeur (13).

8. Procédé pour faire fonctionner un dispositif d'injection selon l'une quelconque des revendications 5 à 7, **caractérisé par** des étapes suivantes :
a. d'insertion du système de transfert de liquide (2) dans le boîtier du dispositif d'injection et de raccordement du système de transfert de liquide (2) à au moins un contenant de liquide,
b. d'établissement d'un raccordement entre le système de transfert de liquide (2) et la conduite en tuyau flexible (18) par l'intermédiaire du dispositif de raccordement,
c. de lecture des données d'identification mémorisées dans le premier transpondeur (22) pour la conduite en tuyau flexible (18) et des données d'identification mémorisées dans le deuxième transpondeur (13) pour le système de transfert de liquide (2) par l'intermédiaire de l'appareil de lecture/d'écriture,
**caractérisé en ce qu'**il est déterminé à l'aide des données lues, avant le début d'un refoulement du liquide à injecter, depuis la mémoire du premier transpondeur (22) et du deuxième transpondeur (13) si et éventuellement à quel moment un refoulement d'un liquide à injecter a eu lieu au préalable pour la première fois en utilisant la conduite en tuyau flexible (18) ou le système de transfert de liquide (2), et que le refoulement débute au moment instantané seulement quand la différence de temps entre le moment instantané et le moment lu de la toute première utilisation est inférieure à une valeur limite prédéfinie.

9. Procédé selon la revendication 8, **caractérisé en ce que** des données sont lues depuis le premier transpondeur (22) et/ou depuis le deuxième transpondeur (13) avant le début d'un refoulement d'un liquide à injecter et les données lues sont comparées à des données de référence, dans lequel le refoulement du liquide à injecter débute alors seulement quand les données lues coïncident avec les données de référence au moins dans une zone prédéfinie.

10. Procédé selon la revendication 8, **caractérisé en ce que** la valeur limite prédéfinie de la différence de temps pour la conduite en tuyau flexible (18) est autre que celle pour le système de transport de liquide (2), dans lequel la valeur limite pour la conduite en tuyau flexible (18) est de manière préférée inférieure à la valeur limite pour le système de transfert de liquide (2).

11. Procédé selon la revendication 8 ou 10, **caractérisé en ce que** le moment d'une toute première utilisation de la conduite en tuyau flexible (18) est inscrit dans une mémoire du premier transpondeur (22) quand il est identifié à l'aide des données lues depuis le premier transpondeur (22) avant le début d'un refoulement d'un liquide à injecter que la conduite en tuyau flexible (18) est utilisée pour la toute première fois, et/ou que le moment de la toute première utilisation du système de transfert de liquide (2) est écrit dans une mémoire du deuxième transpondeur (13) quand il est identifié à l'aide des données lues depuis le deuxième transpondeur avant le début d'un refoulement d'un liquide à injecter que le système de transfert de liquide (1) est utilisé pour la toute première fois.

12. Procédé selon l'une quelconque des revendications 8 à 11, **caractérisé en ce qu'**une durée d'utilisation jusqu'à présent et/ou une durée d'utilisation restante encore admise du système de transfert de liquide (2) et/ou de la conduite en tuyau flexible (18) connectée à celui-ci sont affichées sur une unité d'affichage du dispositif d'injection, et/ou qu'un message d'erreur est affiché sur une unité d'affichage du dispositif d'injection quand les données lues depuis le premier et/ou le deuxième transpondeur (22 ; 13) se situent en dehors d'une plage prédéfinie de valeurs admises ou ne coïncident pas avec des valeurs de référence.
